# EUROPEAN PATENT APPLICATION

(11) **EP 1 321 034 A1**
(43) Date of publication of application: **25.06.2003**
(21) Application number: 02258437.9
(22) Date of filing: 06.12.2002
(51) Int. Cl.: A01K 67/027, C12N 5/10, C12N 9/16, C12N 15/00, C12Q 1/44

(54) **Disruption of the phosphodieterase 10 gene**

(30) Priority: 21.12.2001 US 343478 P
(71) Applicant: Pfizer Products Inc., Groton, Connecticut 06340 (US)
(72) Inventor: Menniti, Frank Samuel, Groton, Connecticut 06340 (US); Schmidt, Christopher Joseph, Groton, Connecticut 06340 (US); Stock, Jeffrey Lynn, Groton, Connecticut 06340 (US); Strick, Christine Ann, Groton, Connecticut 06340 (US)
(74) Representative: England, Peter Michael

(57) **Abstract**

The invention features non-human mammals and animal cells that contain a targeted disruption of a phosphodiesterase 10A (PDE10A) gene.

## Description

This application claims priority, under 35 U.S.C. § 119(e) from U.S. provisional application 60/343,478, which was filed December 21, 2001.

### Field of the Invention

The present invention features genetically-modified non-human mammals and animal cells containing a disrupted phosphodiesterase 10A (PDE10A) gene.

### Background of the Invention

Cyclic nucleotide phosphodiesterases (PDEs) catalyze the hydrolysis of the second messengers cAMP (cyclic adenosine 3'5'-monophosphate) and cGMP (cyclic guanine 3'5'-monophosphate) and play a pivotal regulatory role in a wide variety of signal transduction pathways (Beavo, Physiol. Rev. 75: 725-48, 1995). For example, PDEs mediate processes involved in vision (McLaughlin et al., Nat. Genet. 4: 130-34, 1993), olfaction (Yan et al., Proc. Natl. Acad. Sci. USA 92: 9677-81, 1995), platelet aggregation (Dickinson et al., Biochem. J. 323: 371-77, 1997), aldosterone synthesis (MacFarland et al., J. Biol. Chem. 266: 136-42, 1991), insulin secretion (Zhao et al., J. Clin. Invest. 102: 869-73, 1998), T cell activation (Li et al., Science 283: 848-51, 1999), and smooth muscle relaxation (Boolell et al., Int. J. Impot. Res. 8: 47-52, 1996; Ballard et al., J. Urol. 159: 2164-71, 1998).

PDEs form a superfamily of enzymes that are subdivided into 11 major families (Beavo, Physiol. Rev. 75: 725-48, 1995; Beavo et al., Mol. Pharmacol. 46: 399-05, 1994; Soderling et al., Proc. Natl. Acad. Sci. USA 95: 8991-96, 1998; Fisher et al., Biochem. Biophys. Res. Commun. 246: 570-77, 1998; Hayashi et al., Biochem. Biophys. Res. Commun. 250: 751-56,1998; Soderling et al., J. Biol. Chem. 273: 15553-58, 1998; Fisher et al., J. Biol. Chem. 273: 15559-64, 1998; Soderling et al., Proc. Natl. Acad. Sci. USA 96: 7071-76, 1999; and Fawcett et al., Proc. Natl. Acad. Sci. USA 97: 3702-07, 2000).

The different PDE families are distinguished based on primary sequence homology as well as functionally by unique enzymatic characteristics and pharmacological profiles. In addition, each family exhibits distinct tissue, cellular, and subcellular expression patterns (Beavo et al., Mol. Pharmacol. 46: 399-405, 1994; Soderling et al., Proc. Natl. Acad. Sci. USA 95: 8991-96, 1998; Fisher et al., Biochem. Biophys. Res. Commun. 246: 570-77, 1998; Hayashi et al., Biochem. Biophys. Res. Commun. 250: 751-56, 1998; Soderling et al., J. Biol. Chem. 273: 15553-58, 1998; Fisher et al., J. Biol. Chem. 273: 15559-64, 1998; Soderling et al., Proc. Natl. Acad. Sci. USA 96: 7071-76, 1999; Fawcett et al., Proc. Natl. Acad. Sci. USA 97: 3702-07, 2000; Boolell et al., Int. J. Impot. Res. 8: 47-52, 1996; Ballard et al., J. Urol. 159: 2164-71, 1998; Houslay, Semin. Cell Dev. Biol. 9: 161-67, 1998; and Torphy et al., Pulm. Pharmacol. Ther. 12: 131-35, 1999). Accordingly, by administering a compound that selectively regulates the activity of one family or subfamily of PDE enzymes, it is possible to regulate cAMP and/or cGMP signal transduction pathways in a cell- or tissue-specific manner.

PDE10A is identified as a unique PDE based on primary amino acid sequence and distinct enzymatic activity. Homology screening of EST databases revealed PDE10A as a distinct phosphodiesterase encoded by a single gene (Fujishige et al., J. Biol. Chem. 274: 18438-18445, 1999; Loughney et al., Gene 234:109-117, 1999). The human, rat, and murine homologues of PDE 10 have been cloned and N-terminal splice variants have been identified for both the rat and human genes (Kotera et al., Biochem. Biophys. Res. Comm. 261: 551-557, 1999; Fujishige et al., Eur. J. Biochem. 266: 1118-1127, 1999; Soderling et al., Proc. Natl. Acad. Sci. USA 96: 7071-7076, 1999, U.S. Patent No. 5,932,465, U.S. Pat. No. 6,133,007, EP 0980911A, and U.S. Patent Application No. 60/308,978); there is a high degree of homology across species. PDE10A hydrolyzes cAMP and cGMP to AMP and GMP, respectively. The affinity of PDE10A for cAMP (Kₘ = 0.05 µM) is higher than for cGMP (Kₘ = 3 µM). However, the approximately 5-fold greater Vₘₐₓ for cGMP over cAMP has led to the suggestion that PDE10A is a unique cAMP-inhibited cGMPase (Fujishige et al., J. Biol. Chem. 274:18438-18445, 1999).

PDE10A is uniquely localized in mammals relative to other PDE families. Messenger RNA for PDE10A is most highly expressed in testis and brain (Lanfear and Robas, EP 0967284; Fujishige et al., Eur. J. Biochem. 266: 1118-1127, 1999; Soderling et al., Proc. Natl. Acad. Sci. USA 96: 7071-7076, 1999; Loughney et al., Gene 234:109-117, 1999). Initial studies indicated that, within the brain, expression is highest in the striatum (caudate and putamen, nucleus accumbens, and olfactory tubercle). (Lanfear and Robas, *supra;* U.S. Patent Application No. 60/308,978) Accordingly, PDE10A selective modulation could be used to modulate levels of cyclic nucleotides in these brain areas. Indeed, selective PDE10A inhibition leads to altered basal ganglia function (U.S. Patent Application 60/285,148) and can be effective in treating a variety of neuropsychiatric conditions including psychosis, attention-deficit/hyperactivity disorder (ADHD) and related attentional disorders (Seeman, et al., Molecular Psychiatry 3: 386-96, 1998), depression (Kapur, Biol. Psychiatry 32: 1-17, 1992; Willner, Brain Res. 287: 225-36, 1983), substance abuse (Self, Annals of Med. 30: 379-89, 1998) Parkinson's disease, restless leg syndrome (Hening, Sleep 22: 970-99, 1999), and Huntington's disease.

Additional research tools, including PDE10A knockout mice, would be useful to further define the physiological role of PDE10A action, and the therapeutic implications associated with modulating PDE10A activity.

### Summary of the Invention

The invention features genetically-modified non-human mammals and animal cells that are homozygous or heterozygous for a disrupted PDE10A gene.

In the first aspect, the invention features a genetically-modified, non-human mammal, wherein the modification results in a disrupted PDE10A gene. Preferably, the mammal is a rodent, more preferably, a mouse.

The second aspect of the invention features a genetically-modified animal cell, wherein the modification comprises a disrupted PDE10A gene. In preferred embodiments, the cell is an embryonic stem (ES) cell, an ES-like cell, and/or the cell is murine or human. In another preferred embodiment, the cell is isolated from a genetically-modified, non-human mammal containing a modification that results in a disrupted PDE10A gene. Preferably, the cell is neuronal, e.g., a neuronal stem cell, a neuron in primary culture, or a neuron in a brain slice preparation

In the third aspect, the invention features a method of identifying a gene that demonstrates modified expression as a result of reduced PDE10A activity in an animal cell, the method comprising assessing the expression profile of an animal cell containing a genetic modification that results in reduced PDE10A polypeptide levels in the cell, and comparing that profile to one from a wild type cell. Preferably, the genetically modified animal cell is homozygous for a genetic modification that disrupts the PDE10A gene.

Those skilled in the art will fully understand the terms used herein in the description and the appendant claims to describe the present invention. Nonetheless, unless otherwise provided herein, the following terms are as described immediately below.

A non-human mammal or an animal cell that is "genetically-modified" is heterozygous or homozygous for a modification that is introduced into the non-human mammal or animal cell, or into a progenitor non-human mammal or animal cell, by genetic engineering. The standard methods of genetic engineering that are available for introducing the modification include homologous recombination, viral vector gene trapping, irradiation, chemical mutagenesis, and the transgenic expression of a nucleotide sequence encoding antisense RNA alone or in combination with catalytic ribozymes. Preferred methods for genetic modification to disrupt a gene are those which modify an endogenous gene by inserting a "foreign nucleic acid sequence" into the gene locus, e.g., by homologous recombination or viral vector gene trapping. A "foreign nucleic acid sequence" is an exogenous sequence that is non-naturally occurring in the gene. This insertion of foreign DNA can occur within any region of the PDE10A gene, e.g., in an enhancer, promoter, regulator region, noncoding region, coding region, intron, or exon. The most preferred method of genetic engineering for gene disruption is homologous recombination, in which the foreign nucleic acid sequence is inserted in a targeted manner either alone or in combination with a deletion of a portion of the endogenous gene sequence.

By a PDE10A gene that is "disrupted" is meant a PDE10A gene that is genetically modified such that the cellular activity of the PDE10A polypeptide encoded by the disrupted gene is decreased or eliminated in cells that normally express a wild type version of the PDE10A gene. When the genetic modification effectively eliminates all wild type copies of the PDE10A gene in a cell (e.g., the genetically-modified, non-human mammal or animal cell is homozygous for the PDE10A gene disruption or the only wild type copy of the PDE10A gene originally present is now disrupted), the genetic modification results in a reduction in PDE10A polypeptide activity as compared to a control cell that expresses the wild type PDE10A gene. This reduction in PDE10A polypeptide activity results from either reduced PDE10A gene expression (i.e., PDE10A mRNA levels are effectively reduced resulting in reduced levels of PDE10A polypeptide) and/or because the disrupted PDE10A gene encodes a mutated polypeptide with altered, e.g., reduced, function as compared to a wild type PDE10A polypeptide. Preferably, the activity of PDE10A polypeptide in the genetically-modified, non-human mammal or animal cell is reduced to 50% or less of wild type levels, more preferably, to 25% or less, and, even more preferably, to 10% or less of wild type levels. Most preferably, the PDE10A gene disruption results in a substantial reduction or non-detectable PDE10A activity.

By a "genetically-modified, non-human mammal" containing a disrupted PDE10A gene is meant a non-human mammal that is originally produced, for example, by creating a blastocyst or embryo carrying the desired genetic modification and then implanting the blastocyst or embryo in a foster mother for *in utero* development. The genetically-modified blastocyst or embryo can be made, in the case of mice, by implanting a genetically-modified embryonic stem (ES) cell into a mouse blastocyst or by aggregating ES cells with tetraploid embryos. Alternatively, various species of genetically-modified embryos can be obtained by nuclear transfer. In the case of nuclear transfer, the donor cell is a somatic cell or a pluripotent stem cell, and it is engineered to contain the desired genetic modification that disrupts the PDE10A gene. The nucleus of this cell is then transferred into a fertilized or parthenogenetic oocyte that is enucleated; the resultant embryo is reconstituted and developed into a blastocyst. A genetically-modified blastocyst produced by either of the above methods is then implanted into a foster mother according to standard methods well known to those skilled in the art. A "genetically-modified, non-human mammal" includes all progeny of the non-human mammals created by the methods described above, provided that the progeny inherit at least one copy of the genetic modification that disrupts the PDE10A gene. It is preferred that all somatic cells and germline cells of the genetically-modified non-human mammal contain the modification. Preferred non-human mammals that are genetically-modified to contain a disrupted PDE10A gene include rodents, such as mice and rats, cats, dogs, rabbits, guinea pigs, hamsters, sheep, pigs, and ferrets.

By a "genetically-modified animal cell" containing a disrupted PDE10A gene is meant an animal cell, including a human cell, created by genetic engineering to contain a disrupted PDE10A gene, as well as daughter cells that inherit the disrupted PDE10A gene. These cells may be genetically-modified in culture according to any standard method known in the art. As an alternative to genetically modifying the cells in culture, non-human mammalian cells may also be isolated from a genetically-modified, non-human mammal that contains a PDE10A gene disruption. The animal cells of the invention may be obtained from primary cell or tissue preparations as well as culture-adapted, tumorigenic, or transformed cell lines. These cells and cell lines are derived, for example, from endothelial cells, epithelial cells, islets, neurons and other neural tissue-derived cells, mesothelial cells, osteocytes, lymphocytes, chondrocytes, hematopoietic cells, immune cells, cells of the major glands or organs (e.g., testicle, liver, lung, heart, stomach, pancreas, kidney, and skin), muscle cells (including cells from skeletal muscle, smooth muscle, and cardiac muscle), exocrine or endocrine cells, fibroblasts, and embryonic and other totipotent or pluripotent stem cells (e.g., ES cells, ES-like cells, and embryonic germline (EG) cells, and other stem cells, such as progenitor cells and tissue-derived stem cells). The preferred genetically-modified cells are ES cells, more preferably, mouse or rat ES cells, and, most preferably, human ES cells.

By "PDE10A activity" is meant PDE10A-mediated hydrolysis of cAMP and/or cGMP.

By "reduced PDE10A activity" is meant a decrease in the activity of the PDE10A enzyme as a result of genetic manipulation of the PDE10A gene that causes a reduction in the level of PDE10A polypeptide in a cell, or as the result of administration of a pharmacological agent that inhibits PDE10A activity.

By an "ES cell" or an "ES-like cell" is meant a pluripotent stem cell derived from an embryo, from a primordial germ cell, or from a teratocarcinoma, that is capable of indefinite self renewal as well as differentiation into cell types that are representative of all three embryonic germ layers.

Other features and advantages of the invention will be apparent from the following detailed description and from the claims. While the invention is described in connection with specific embodiments, it will be understood that other changes and modifications that may be practiced are also part of this invention and are also within the scope of the appendant claims. This application is intended to cover any equivalents, variations, uses, or adaptations of the invention that follow, in general, the principles of the invention, including departures from the present disclosure that come within known or customary practice within the art, and that are able to be ascertained without undue experimentation. Additional guidance with respect to making and using nucleic acids and polypeptides is found in standard textbooks of molecular biology, protein science, and immunology (see, e.g., Davis et al., *Basic Methods in Molecular Biology,* Elsevir Sciences Publishing, Inc., New York, NY, 1986; Hames et al., *Nucleic Acid Hybridization*, IL Press, 1985; Molecular Cloning, Sambrook et al., *Current Protocols in Molecular Biology,* Eds. Ausubel et al., John Wiley and Sons; *Current Protocols in Human Genetics,* Eds. Dracopoli et al., John Wiley and Sons; *Current Protocols in Protein Science,* Eds. John E. Coligan et al., John Wiley and Sons; and *Current Protocols in Immunology,* Eds. John E. Coligan et al., John Wiley and Sons). All publications mentioned herein are incorporated by reference in their entireties.

### Brief Description of the Figures

Fig. 1 is a schematic depicting the PDE10A (PDE10) gene targeting vector, the location for homologous recombination of the vector in the endogenous murine PDE10A gene, and the Southern blot strategy used to verify gene targeting.
Fig. 2 shows the Southern analysis of ES Cells. Clone #176 contains the targeted allele as well as the endogenous allele.
Fig. 3 shows the results of polymerase chain reaction (PCR)-based genotyping of heterozygote (+/-) and knockout (-/-) mice with respect to the disrupted PDE10A allele.
Fig. 4 shows a Western blot using a monoclonal antibody (24F3.F11) to the N-terminal region of PDE10A. Approximate molecular weight markers are on the right. In Lane 1 is purified recombinant rat PDE10A, which is seen as a single band running at molecular weight range of about 89 kDa. Lanes 2-4 are 10 ug striatal extracts from PDE10A *+*/*+, +*/-*,* or -/-. The band running at approximately 66 kDa in the striatal extracts is not a phosphodiesterase or related to PDE10A.
Fig. 5A confirms the relative phosphodiesterase activity (indicated on the X-axis as an increase in the accumulation of [³H]5'-AMP (in dpm/well), the product of phosphodiesterase catalyzed breakdown of cAMP) of Sf9 cell extracts (Control), extracts from Sf9 cells transfected with cDNA coding full length PDE10A (PDE10A) or, Sf9 cells transfected with PDE10A cDNA isolated from PDE10 KO mouse brain. Note that the concentration of extract from PDE10A transfected cells is 100-fold lower than that for untransfected cells or the mutant PDE10A transfected cells.
Fig. 5B shows the relative phosphodiesterase activity (indicated on the X-axis as an increase in the accumulation of [³H]5'-AMP (in dpm/well), the product of phosphodiesterase catalyzed breakdown of cAMP) in equivalent amounts of immunoprecipitates from striatum (black bars) or cortex (gray bars) from PDE10A +/+, +/-, or -/- mice. Each bar represents the mean from 2 animals.
Fig. 6 shows cGMP levels in extracts from striatum (left) or cortex (right) from PDE10A +/+ or -/- mice treated with 32 mg/kg papaverine (black bars) or vehicle (gray bars). Each bar represents the mean ± SEM from 5 animals.
Fig. 7 shows the mean number of avoidances for PDE10A +/+ (black bars) or -/- (gray bars) mice treated with the indicated doses of papaverine. Each bar represents the mean ± SEM from 4 animals in which each animal received each of the indicated doses on different days. ** indicates statistical difference from the vehicle treated value (0 Papaverine).

### Detailed Description of the Invention

### Genetically-Modified Non-human Mammals and Animal Cells Containing a Disrupted PDE10A Gene

### 1. Genetically-Modified Non-human Mammals and Animal Cells

The genetically-modified, non-human mammals and genetically-modified animal cells, including human cells, of the invention are heterozygous or homozygous for a modification that disrupts the PDE10A gene. The animal cells may be derived by genetically engineering cells in culture, or, in the case of non-human mammalian cells, the cells may be isolated from genetically-modified, non-human mammals.

The PDE10A gene locus is disrupted by one of the several techniques for genetic modification known in the art, including chemical mutagenesis (Rinchik, Trends in Genetics 7: 15-21, 1991, Russell, Environmental & Molecular Mutagenesis 23 (Suppl. 24): 23-29, 1994), irradiation (Russell, *supra),* transgenic expression of PDE10A gene antisense RNA, either alone or in combination with a catalytic RNA ribozyme sequence (Luyckx et al., Proc. Natl. Acad. Sci. 96: 12174-79, 1999; Sokol et al., Transgenic Research 5: 363-71, 1996; Efrat et al., Proc. Natl. Acad. Sci. USA 91: 2051-55, 1994; Larsson et al., Nucleic Acids Research 22: 2242-48, 1994) and, as further discussed below, the disruption of the PDE10A gene by the insertion of a foreign nucleic acid sequence into the PDE10A gene locus. Preferably, the foreign sequence is inserted by homologous recombination or by the insertion of a viral vector. Most preferably, the method of PDE10A gene disruption to create the genetically modified non-human mammals and animal cells of the invention is homologous recombination and includes a deletion of a portion of the endogenous PDE10A gene sequence.

The integration of the foreign sequence disrupts the PDE10A gene through one or more of the following mechanisms: by interfering with the PDE10A gene transcription or translation process (e.g., by interfering with promoter recognition, or by introducing a transcription termination site or a translational stop codon into the PDE10A gene); or by distorting the PDE10A gene coding sequence such that it no longer encodes a PDE10A polypeptide with normal function (e.g., by inserting a foreign coding sequence into the PDE10A gene coding sequence, by introducing a frameshift mutation or amino acid(s) substitution, or, in the case of a double crossover event, by deleting a portion of the PDE10A gene coding sequence that is required for expression of a functional PDE10A protein).

To insert a foreign sequence into a PDE10A gene locus in the genome of a cell to create the genetically modified non-human mammals and animal cells of the invention based upon the present description, the foreign DNA sequence is introduced into the cell according to a standard method known in the art such as electroporation, calcium-phosphate precipitation, retroviral infection, microinjection, biolistics, liposome transfection, DEAE-dextran transfection, or transfection (see, e.g., Neumann et al., EMBO J. 1: 841-845, 1982; Potter et al., Proc. Natl. Acad. Sci USA 81: 7161-65, 1984; Chu et al., Nucleic Acids Res. 15: 1311-26, 1987; Thomas and Capecchi, Cell 51: 503-12, 1987; Baum et al., Biotechniques 17: 1058-62, 1994; Biewenga et al., J. Neuroscience Methods 71: 67-75, 1997; Zhang et al., Biotechniques 15: 868-72, 1993; Ray and Gage, Biotechniques 13: 598-603, 1992; Lo, Mol. Cell. Biol. 3: 1803-14, 1983; Nickoloff et al., Mol. Biotech. 10: 93-101, 1998; Linney et al., Dev. Biol. (Orlando) 213: 207-16, 1999; Zimmer and Gruss, Nature 338: 150-153, 1989; and Robertson et al., Nature 323: 445-48, 1986). The preferred method for introducing foreign DNA into a cell is electroporation.

### 2. Homologous Recombination

The method of homologous recombination targets the PDE10A gene for disruption by introducing a PDE10A gene targeting vector into a cell containing a PDE10A gene. The ability of the vector to target the PDE10A gene for disruption stems from using a nucleotide sequence in the vector that is homologous, i.e., related, to the PDE10A gene. This homology region facilitates hybridization between the vector and the endogenous sequence of the PDE10A gene. Upon hybridization, the probability of a crossover event between the targeting vector and genomic sequences greatly increases. This crossover event results in the integration of the vector sequence into the PDE10A gene locus and the functional disruption of the PDE10A gene.

General principles regarding the construction of vectors used for targeting are reviewed in Bradley et al. (Biotechnol. 10: 534, 1992). Two different types of vector can be used to insert DNA by homologous recombination: an insertion vector or a replacement vector. An insertion vector is circular DNA which contains a region of PDE10A gene homology with a double stranded break. Following hybridization between the homology region and the endogenous PDE10A gene, a single crossover event at the double stranded break results in the insertion of the entire vector sequence into the endogenous gene at the site of crossover.

The more preferred vector to create the genetically modified non-human mammals and animals cells of the invention by homologous recombination is a replacement vector, which is colinear rather than circular. Replacement vector integration into the PDE10A gene requires a double crossover event, i.e. crossing over at two sites of hybridization between the targeting vector and the PDE10A gene. This double crossover event results in the integration of a vector sequence that is sandwiched between the two sites of crossover into the PDE10A gene and the deletion of the corresponding endogenous PDE10A gene sequence that originally spanned between the two sites of crossover (see, e.g., Thomas and Capecchi et al., Cell 51: 503-12, 1987; Mansour et al., Nature 336: 348-52, 1988; Mansour et al., Proc. Natl. Acad. Sci. USA 87: 7688-7692, 1990; and Mansour, GATA 7: 219-227, 1990).

A region of homology in a targeting vector used to create the genetically modified non-human mammals and animal cells of the invention is generally at least 100 nucleotides in length. Most preferably, the homology region is at least 1-5 kilobases (kb) in length. Although there is no demonstrated minimum length or minimum degree of relatedness required for a homology region, targeting efficiency for homologous recombination generally corresponds with the length and the degree of relatedness between the targeting vector and the PDE10A gene locus. In the case where a replacement vector is used, and a portion of the endogenous PDE10A gene is deleted upon homologous recombination, an additional consideration is the size of the deleted portion of the endogenous PDE10A gene. If this portion of the endogenous PDE10A gene is greater than 1 kb in length, then a targeting cassette with regions of homology that are longer than 1 kb is recommended to enhance the efficiency of recombination. Further guidance regarding the selection and use of sequences effective for homologous recombination, based on the present description, is described in the literature (see, e.g., Deng and Capecchi, Mol. Cell. Biol. 12: 3365-3371, 1992; Bollag et al., Annu. Rev. Genet. 23: 199-225, 1989; and Waldman and Liskay, Mol. Cell. Biol. 8: 5350-5357, 1988).

As those skilled in the art will recognize based upon the present invention, a wide variety of cloning vectors may be used as vector backbones in the construction of the PDE10A gene targeting vectors of the present invention, including pBluescript-related plasmids (e.g., Bluescript KS+11), pQE70, pQE60, pQE-9, pBS, pD10, phagescript, phiX174, pBK Phagemid, pNH8A, pNH16a, pNH18Z, pNH46A, ptrc99a, pKK223-3, pKK233-3, pDR540, and pRIT5 PWLNEO, pSV2CAT, pXT1, pSG (Stratagene), pSVK3, PBPV, PMSG, and pSVL, pBR322 and pBR322-based vectors, pMB9, pBR325, pKH47, pBR328, pHC79, phage Charon 28, pKB11, pKSV-10, pK19 related plasmids, pUC plasmids, and the pGEM series of plasmids. These vectors are available from a variety of commercial sources (e.g., Boehringer Mannheim Biochemicals, Indianapolis, IN; Qiagen, Valencia, CA; Stratagene, La Jolla, CA; Promega, Madison, WI; and New England Biolabs, Beverly, MA). However, any other vectors, e.g. plasmids, viruses, or parts thereof, may be used as long as they are replicable and viable in the desired host. The vector may also comprise sequences which enable it to replicate in the host whose genome is to be modified. The use of such a vector can expand the interaction period during which recombination can occur, increasing the efficiency of targeting (see *Molecular Biology,* ed. Ausubel et al, Unit 9.16, Fig. 9.16.1).

The specific host employed for propagating the targeting vectors of the present invention is not critical. Examples include *E. coli* K12 RR1 (Bolivar et al., Gene 2: 95, 1977), *E. coli* K12 HB101 (ATCC No. 33694), *E. coli* MM21 (ATCC No. 336780), *E. coli* DH1 (ATCC No. 33849), *E. coli* strain DH5*a*, and *E. coli* STBL2. Alternatively, hosts such as *C. cerevisiae* or *B. subtilis* can be used. The above-mentioned hosts are available commercially (e.g., Stratagene, La Jolla, CA; and Life Technologies, Rockville, MD).

To create the targeting vector, a PDE10A gene targeting construct is added to an above-described vector backbone. The PDE10A gene targeting constructs of the invention have at least one PDE10A gene homology region. To make the PDE10A gene homology regions, a PDE10A genomic or cDNA sequence is used as a basis for producing PCR primers. These primers are used to amplify the desired region of the PDE10A sequence by high fidelity PCR amplification (Mattila et al., Nucleic Acids Res. 19: 4967, 1991; Eckert and Kunkel 1: 17, 1991; and U.S. Pat. No. 4,683, 202). The genomic sequence is obtained from a genomic clone library or from a preparation of genomic DNA, preferably from the animal species that is to be targeted for PDE10A gene disruption. The PDE10A cDNA sequence can be used in making a PDE10A targeting vector (e.g. murine, Genbank AF110507; human, Genbank AB020593; rat, EP 0980911A; U.S. Pat. Application No. 60/308,978).

Preferably, the targeting constructs of the invention also include an exogenous nucleotide sequence encoding a positive marker protein. The stable expression of a positive marker after vector integration confers an identifiable characteristic on the cell, ideally, without compromising cell viability. Therefore, in the case of a replacement vector, the marker gene is positioned between two flanking homology regions so that it integrates into the PDE10A gene following the double crossover event in a manner such that the marker gene is positioned for expression after integration.

It is preferred that the positive marker protein is a selectable protein; the stable expression of such a protein in a cell confers a selectable phenotypic characteristic, i.e., the characteristic enhances the survival of the cell under otherwise lethal conditions. Thus, by imposing the selectable condition, one can isolate cells that stably express the positive selectable marker-encoding vector sequence from other cells that have not successfully integrated the vector sequence on the basis of viability. Examples of positive selectable marker proteins (and their agents of selection) include neo (G418 or kanomycin), hyg (hygromycin), hisD (histidinol), gpt (xanthine), ble (bleomycin), and hprt (hypoxanthine) (see, e.g., Capecchi and Thomas, U.S. Pat. No. 5,464,764, and Capecchi, Science 244: 1288-92, 1989). Other positive markers that may also be used as an alternative to a selectable marker include reporter proteins such as β-galactosidase, firefly luciferase, or GFP (see, e.g., *Current Protocols in Cytometry,* Unit 9.5, and *Current Protocols in Molecular Biology,* Unit 9.6, John Wiley & Sons, New York, NY, 2000).

The above-described positive selection step does not distinguish between cells that have integrated the vector by targeted homologous recombination at the PDE10A gene locus versus random, non-homologous integration of vector sequence into any chromosomal position. Therefore, when using a replacement vector for homologous recombination to make the genetically modified non-human mammals and animal cells of the invention, it is also preferred to include a nucleotide sequence encoding a negative selectable marker protein. Expression of a negative selectable marker causes a cell expressing the marker to lose viability when exposed to a certain agent (i.e., the marker protein becomes lethal to the cell under certain selectable conditions). Examples of negative selectable markers (and their agents of lethality) include herpes simplex virus thymidine kinase (gancyclovir or 1,2-deoxy-2-fluoro-α-d-arabinofuransyl-5-iodouracil), Hprt (6-thioguanine or 6-thioxanthine), and diphtheria toxin, ricin toxin, and cytosine deaminase (5-fluorocytosine).

The nucleotide sequence encoding the negative selectable marker is positioned outside of the two homology regions of the replacement vector. Given this positioning, cells will only integrate and stably express the negative selectable marker if integration occurs by random, non-homologous recombination; homologous recombination between the PDE10A gene and the two regions of homology in the targeting construct excludes the sequence encoding the negative selectable marker from integration. Thus, by imposing the negative condition, cells that have integrated the targeting vector by random, non-homologous recombination lose viability.

The above-described combination of positive and negative selectable markers is preferred in a targeting construct used to make the genetically modified non-human mammals and animal cells of the invention because a series of positive and negative selection steps can be designed to more efficiently select only those cells that have undergone vector integration by homologous recombination, and, therefore, have a potentially disrupted PDE10A gene. Further examples of positive-negative selection schemes, selectable markers, and targeting constructs are described, for example, in U.S. Pat. No. 5,464,764, WO 94/06908, U.S. Pat. No. 5,859,312, and Valancius and Smithies, Mol. Cell. Biol. 11: 1402, 1991.

In order for a marker protein to be stably expressed upon vector integration, the targeting vector may be designed so that the marker coding sequence is operably linked to the endogenous PDE10A gene promoter upon vector integration. Expression of the marker is then driven by the PDE10A gene promoter in cells that normally express the PDE10A gene. Alternatively, each marker in the targeting construct of the vector may contain its own promoter that drives expression independent of the PDE10A gene promoter. This latter scheme has the advantage of allowing for expression of markers in cells that do not typically express the PDE10A gene (Smith and Berg, Cold Spring Harbor Symp. Quant. Biol. 49: 171, 1984; Sedivy and Sharp, Proc. Natl. Acad. Sci. (USA) 86: 227, 1989; Thomas and Capecchi, Cell 51: 503, 1987).

Exogenous promoters that can be used to drive marker gene expression include cell-specific or stage-specific promoters, constitutive promoters, and inducible or regulatable promoters. Non-limiting examples of these promoters include the herpes simplex thymidine kinase promoter, cytomegalovirus (CMV) promoter/enhancer, SV40 promoters, PGK promoter, PMC1-neo, metallothionein promoter, adenovirus late promoter, vaccinia virus 7.5K promoter, avian beta globin promoter, histone promoters (e.g., mouse histone H3-614), beta actin promoter, neuron-specific enolase, muscle actin promoter, and the cauliflower mosaic virus 35S promoter (see generally, Sambrook et al., *Molecular Cloning,* Vols. I-III, Cold Spring Harbor Laboratory Press, Cold Spring Harbor, NY, 1989, and *Current Protocols in Molecular Biology,* John Wiley & Sons, New York, NY, 2000; Stratagene, La Jolla, CA).

To confirm whether cells have integrated the vector sequence into the targeted PDE10A gene locus while making the genetically modified non-human mammals and animal cells of the invention, primers or genomic probes that are specific for the desired vector integration event can be used in combination with PCR or Southern blot analysis to identify the presence of the desired vector integration into the PDE10A gene locus (Erlich et al., Science 252: 1643-51, 1991; Zimmer and Gruss, Nature 338: 150, 1989; Mouellic et al., Proc. Natl. Acad. Sci. (USA) 87: 4712, 1990; and Shesely et al., Proc. Natl. Acad. Sci. (USA) 88: 4294, 1991).

### 3. Gene Trapping

Another method available for inserting a foreign nucleic acid sequence into the PDE10A gene locus to disrupt the PDE10A gene, based on the present description, is gene trapping. This method takes advantage of the cellular machinery present in all mammalian cells that splices exons into mRNA to insert a gene trap vector coding sequence into a gene in a random fashion. Once inserted, the gene trap vector creates a mutation that may disrupt the trapped PDE10A gene. In contrast to homologous recombination, this system for mutagenesis creates largely random mutations. Thus, to obtain a genetically-modified cell that contains a disrupted PDE10A gene, cells containing this particular mutation must be identified and selected from a pool of cells that contain random mutations in a variety of genes.

Gene trapping systems and vectors have been described for use in genetically modifying murine cells and other cell types (see, e.g., Allen et al., Nature 333: 852-55, 1988; Bellen et al., Genes Dev. 3: 1288-1300, 1989; Bier et al., Genes Dev. 3: 1273-1287, 1989; Bonnerot et al., J. Virol. 66: 4982-91, 1992; Brenner et al., Proc. Nat. Acad. Sci. USA 86: 5517-21, 1989; Chang et al., Virology 193: 737-47, 1993; Friedrich and Soriano, Methods Enzymol. 225: 681-701, 1993; Friedrich and Soriano, Genes Dev. 5: 1513-23, 1991; Goff, Methods Enzymol. 152: 469-81, 1987; Gossler et al., Science 244: 463-65, 1989; Hope, Develop. 113: 399-408, 1991; Kerr et al., Cold Spring Harb. Symp. Quant. Biol. 2: 767-776, 1989; Reddy et al., J. Virol. 65: 1507-1515, 1991; Reddy et al., Proc. Natl. Acad. Sci. U.S.A. 89: 6721-25, 1992; Skarnes et al., Genes Dev. 6: 903-918, 1992; von Melchner and Ruley, J. Virol. 63: 3227-3233, 1989; and Yoshida et al., Transgen. Res. 4: 277-87, 1995).

Promoter trap, or 5', vectors contain, in 5' to 3' order, a splice acceptor sequence followed by an exon, which is typically characterized by a translation initiation codon and open reading frame and/or an internal ribosome entry site. In general, these promoter trap vectors do not contain promoters or operably linked splice donor sequences. Consequently, after integration into the cellular genome of the host cell, the promoter trap vector sequence intercepts the normal splicing of the upstream gene and acts as a terminal exon. Expression of the vector coding sequence is dependent upon the vector integrating into an intron of the disrupted gene in the proper reading frame. In such a case, the cellular splicing machinery splices exons from the trapped gene upstream of the vector coding sequence (Zambrowicz et al., WO 99/50426 and U.S. Pat. No. 6,080,576).

An alternative method for producing an effect similar to the above-described promoter trap vector is a vector that incorporates a nested set of stop codons present in, or otherwise engineered into, the region between the splice acceptor of the promoter trap vector and the translation initiation codon or polyadenylation sequence. The coding sequence can also be engineered to contain an independent ribosome entry site (IRES) so that the coding sequence will be expressed in a manner largely independent of the site of integration within the host cell genome. Typically, but not necessarily, an IRES is used in conjunction with a nested set of stop codons.

Another type of gene trapping scheme uses a 3' gene trap vector. This type of vector contains, in operative combination, a promoter region, which mediates expression of an adjoining coding sequence, the coding sequence, and a splice donor sequence that defines the 3' end of the coding sequence exon. After integration into a host cell genome, the transcript expressed by the vector promoter is spliced to a splice acceptor sequence from the trapped gene that is located downstream of the integrated gene trap vector sequence. Thus, the integration of the vector results in the expression of a fusion transcript comprising the coding sequence of the 3' gene trap cassette and any downstream cellular exons, including the terminal exon and its polyadenylation signal. When such vectors integrate into a gene, the cellular splicing machinery splices the vector coding sequence upstream of the 3' exons of the trapped gene. One advantage of such vectors is that the expression of the 3' gene trap vectors is driven by a promoter within the gene trap cassette and does not require integration into a gene that is normally expressed in the host cell (Zambrowicz et al., WO 99/50426 and U.S. Pat. No. 6,080,576). Examples of transcriptional promoters and enhancers that may be incorporated into the 3' gene trap vector include those discussed above with respect to targeting vectors.

The viral vector backbone used as the structural component for the promoter or 3' gene trap vector may be selected from a wide range of vectors that can be inserted into the genome of a target cell. Suitable backbone vectors include, but are not limited to, herpes simplex virus vectors, adenovirus vectors, adeno-associated virus vectors, retroviral vectors, lentiviral vectors, pseudorabies virus, alpha-herpes virus vectors, and the like. A thorough review of viral vectors, in particular, viral vectors suitable for modifying nonreplicating cells and how to use such vectors in conjunction with the expression of an exogenous polynucleotide sequence, can be found in *Viral Vectors: Gene Therapy and Neuroscience Applications*, Eds. Caplitt and Loewy, Academic Press, San Diego, 1995.

Preferably, retroviral vectors are used for gene trapping. These vectors can be used in conjunction with retroviral packaging cell lines such as those described in U.S. Patent No. 5,449,614. Where non-murine mammalian cells are used as target cells for genetic modification, amphotropic or pantropic packaging cell lines can be used to package suitable vectors (Ory et al., Proc. Natl. Acad. Sci., USA 93: 11400-11406, 1996). Representative retroviral vectors that can be adapted to create the presently described 3' gene trap vectors are described, for example, in U.S. Pat. No. 5,521,076.

The gene trapping vectors may contain one or more of the positive marker genes discussed above with respect to targeting vectors used for homologous recombination. Similar to their use in targeting vectors, these positive markers are used in gene trapping vectors to identify and select cells that have integrated the vector into the cell genome. The marker gene may be engineered to contain an independent ribosome entry site (IRES) so that the marker will be expressed in a manner largely independent of the location in which the vector has integrated into the target cell genome.

Given that gene trap vectors will integrate into the genome of infected host cells in a fairly random manner, a genetically-modified cell having a disrupted PDE10A gene must be identified from a population of cells that have undergone random vector integration. Preferably, the genetic modifications in the population of cells are of sufficient randomness and frequency such that the population represents mutations in essentially every gene found in the cell's genome, making it likely that a cell with a disrupted PDE10A gene will be identified from the population (see Zambrowicz et al., WO 99/50426; Sands et al., WO 98/14614 and U.S. Pat. No. 6,080,576).

Individual mutant cell lines containing a disrupted PDE10A gene are identified in a population of mutated cells using, for example, reverse transcription and polymerase chain reaction (PCR) to identify a mutation in a PDE10A gene sequence. This process can be streamlined by pooling clones. For example, to find an individual clone containing a disrupted PDE10A gene, RT-PCR is performed using one primer anchored in the gene trap vector and the other primer located in the PDE10A gene sequence. A positive RT-PCR result indicates that the vector sequence is encoded in the PDE10A gene transcript, indicating that PDE10A gene has been disrupted by a gene trap integration event (see, e.g., Sands et al., WO 98/14614, U.S. Pat. No. 6,080,576).

### 4. Temporal, Spatial, and Inducible PDE10A Gene Disruptions

In certain embodiments of the present invention, a functional disruption of the endogenous PDE10A gene occurs at specific developmental or cell cycle stages (temporal disruption) or in specific cell types (spatial disruption). In other embodiments, the PDE10A gene disruption is inducible when certain conditions are present. A recombinase excision system, such as a Cre-Lox system, may be used to activate or inactivate the PDE10A gene at a specific developmental stage, in a particular tissue or cell type, or under particular environmental conditions. Generally, methods utilizing Cre-Lox technology are carried out as described by Torres and Kuhn, *Laboratory Protocols for Conditional Gene Targeting,* Oxford University Press, 1997. Methodology similar to that described for the Cre-Lox system can also be employed utilizing the FLP-FRT system. Further guidance regarding the use of recombinase excision systems for conditionally disrupting genes by homologous recombination or viral insertion is provided, for example, in U.S. Pat. No. 5,626,159, U.S. Pat. No. 5,527,695, U.S. Pat. No. 5,434,066, WO 98/29533, U.S. Pat. No. 6,228,639, Orban et al., Proc. Nat. Acad. Sci. USA 89: 6861-65, 1992; O'Gorman et al., Science 251: 1351-55, 1991; Sauer et al., Nucleic Acids Research 17: 147-61, 1989; Barinaga, Science 265: 26-28, 1994; and Akagi et al., Nucleic Acids Res. 25: 1766-73, 1997. More than one recombinase system can be used to genetically modify a non-human mammal or animal cell of the present invention.

When using homologous recombination to disrupt the PDE10A gene in a temporal, spatial, or inducible fashion, using a recombinase system such as the Cre-Lox system, a portion of the PDE10A gene coding region is replaced by a targeting construct comprising the PDE10A gene coding region flanked by loxP sites. Non-human mammals and animal cells carrying this genetic modification contain a functional, loxP-flanked PDE10A gene. The temporal, spatial, or inducible aspect of the PDE10A gene disruption is caused by the expression pattern of an additional transgene, a Cre recombinase transgene, that is expressed in the non-human mammal or animal cell under the control of the desired spatially-regulated, temporally-regulated, or inducible promoter, respectively. A Cre recombinase targets the loxP sites for recombination. Therefore, when Cre expression is activated, the LoxP sites undergo recombination to excise the sandwiched PDE10A gene coding sequence, resulting in a functional disruption of the PDE10A gene (Rajewski et al., J. Clin. Invest. 98: 600-03, 1996; St.-Onge et al., Nucleic Acids Res. 24: 3875-77, 1996; Agah et al., J. Clin. Invest. 100: 169-79, 1997; Brocard et al., Proc. Natl. Acad. Sci. USA 94: 14559-63, 1997; Feil et al., Proc. Natl. Acad. Sci. USA 93: 10887-90, 1996; and Kühn et al., Science 269: 1427-29, 1995).

A cell containing both a Cre recombinase transgene and loxP-flanked PDE10A gene can be generated through standard transgenic techniques or, in the case of genetically-modified, non-human mammals, by crossing genetically-modified, non-human mammals wherein one parent contains a loxP flanked PDE10A gene and the other contains a Cre recombinase transgene under the control of the desired promoter. Further guidance regarding the use of recombinase systems and specific promoters to temporally, spatially, or conditionally disrupt the PDE10A gene is found, for example, in Sauer, Meth. Enz. 225: 890-900, 1993, Gu et al., Science 265: 103-06, 1994, Araki et al., J. Biochem. 122: 977-82, 1997, Dymecki, Proc. Natl. Acad. Sci. 93: 6191-96, 1996, and Meyers et al., Nature Genetics 18: 136-41, 1998.

An inducible disruption of the PDE10A gene can also be achieved by using a tetracycline responsive binary system (Gossen and Bujard, Proc. Natl. Acad. Sci. USA 89: 5547-51, 1992). This system involves genetically modifying a cell to introduce a Tet promoter into the endogenous PDE10A gene regulatory element and a transgene expressing a tetracycline-controllable repressor (TetR). In such a cell, the administration of tetracycline activates the TetR which, in turn, inhibits PDE10A gene expression and, therefore, disrupts the PDE10A gene (St.-Onge et al., Nucleic Acids Res. 24: 3875-77, 1996, U.S. Patent No. 5,922,927).

The above-described systems for temporal, spatial, and inducible disruptions of the PDE10A gene can also be adopted when using gene trapping as the method of genetic modification, for example, as described, in WO 98/29533 and U.S. Pat. No. 6,288,639, for creating the genetically modified non-human mammals and animal cells of the invention.

### 5. Creating Genetically-Modified, Non-human Mammals and Animal Cells

The above-described methods for genetic modification can be used to disrupt a PDE10A gene in virtually any type of somatic or stem cell derived from an animal to create the genetically modified animal cells of the invention. Genetically-modified animal cells of the invention include, but are not limited to, mammalian cells, including human cells, and avian cells. These cells may be derived from genetically engineering any animal cell line, such as culture-adapted, tumorigenic, or transformed cell lines, or they may be isolated from a genetically-modified, non-human mammal carrying the desired PDE10A genetic modification.

The cells may be heterozygous or homozygous for the disrupted PDE10A gene. To obtain cells that are homozygous for the PDE10A gene disruption (PDE10-/-), direct, sequential targeting of both alleles can be performed. This process can be facilitated by recycling a positive selectable marker. According to this scheme the nucleotide sequence encoding the positive selectable marker is removed following the disruption of one allele using the Cre-Lox P system. Thus, the same vector can be used in a subsequent round of targeting to disrupt the second PDE10A gene allele (Abuin and Bradley, Mol. Cell. Biol. 16: 1851-56, 1996; Sedivy et al., T.I.G. 15: 88-90, 1999; Cruz et al., Proc. Natl. Acad. Sci. (USA) 88: 7170-74, 1991; Mortensen et al., Proc. Natl. Acad. Sci. (USA) 88: 7036-40, 1991; te Riele et al., Nature (London) 348: 649-651, 1990).

An alternative strategy for obtaining ES cells that are PDE10-/- is the homogenotization of cells from a population of cells that is heterozygous for the PDE10A gene disruption (PDE10+/-). The method uses a scheme in which PDE10+/- targeted clones that express a selectable drug resistance marker are selected against a very high drug concentration; this selection favors cells that express two copies of the sequence encoding the drug resistance marker and are, therefore, homozygous for the PDE10A gene disruption (Mortensen et al., Mol. Cell. Biol. 12: 2391-95, 1992). In addition, genetically-modified animal cells can be obtained from genetically-modified PDE10-/- non-human mammals that are created by mating non-human mammals that are PDE10+/- in germline cells, as further discussed below.

Following the genetic modification of the desired cell or cell line, the PDE10A gene locus can be confirmed as the site of modification by PCR analysis according to standard PCR or Southern blotting methods known in the art (see, e.g., U.S. Pat. No. 4,683,202; and Erlich et al., Science 252: 1643, 1991). Further verification of the functional disruption of the PDE10A gene may also be made if PDE10A gene messenger RNA (mRNA) levels and/or PDE10A polypeptide levels are reduced in cells that normally express the PDE10A gene. Measures of PDE10A gene mRNA levels may be obtained by using reverse transcriptase mediated polymerase chain reaction (RT-PCR), Northern blot analysis, or *in situ* hybridization. The quantification of PDE10A polypeptide levels produced by the cells can be made, for example, by standard immunoassay methods known in the art. Such immunoassays include, but are not limited to, competitive and non-competitive assay systems using techniques such as RIAs (radioimmunoassays), ELISAs (enzyme-linked immunosorbent assays), "sandwich" immunoassays, immunoradiometric assays, gel diffusion precipitin reactions, immunodiffusion assays, *in situ* immunoassays (using, for example, colloidal gold, enzymatic, or radioisotope labels), Western blots, 2-dimensional gel analysis, precipitation reactions, immunofluorescence assays, protein A assays, and immunoelectrophoresis assays.

Preferred genetically-modified animal cells of the invention are embryonic stem (ES) cells and ES-like cells. These cells are derived from the preimplantation embryos and blastocysts of various species, such as mice (Evans et al., Nature 129:154-156, 1981; Martin, Proc. Natl. Acad. Sci., USA, 78: 7634-7638, 1981), pigs and sheep (Notanianni et al., J. Reprod. Fert. Suppl., 43: 255-260, 1991; Campbell et al., Nature 380: 64-68,1996) and primates, including humans (Thomson et al., U.S. Patent No. 5,843,780, Thomson et al., Science 282: 1145-1147, 1995; and Thomson et al., Proc. Natl. Acad. Sci. USA 92: 7844-7848, 1995).

These types of cells are pluripotent, that is, under proper conditions, they differentiate into a wide variety of cell types derived from all three embryonic germ layers: ectoderm, mesoderm and endoderm. Depending upon the culture conditions, a sample of ES cells can be cultured indefinitely as stem cells, allowed to differentiate into a wide variety of different cell types within a single sample, or directed to differentiate into a specific cell type, such as macrophage-like cells, neuronal cells, cardiomyocytes, chondrocytes, adipocytes, smooth muscle cells, endothelial cells, skeletal muscle cells, keratinocytes, and hematopoietic cells, such as eosinophils, mast cells, erythroid progenitor cells, or megakaryocytes. Directed differentiation is accomplished by including specific growth factors or matrix components in the culture conditions, as further described, for example, in Keller et al., Curr. Opin. Cell Biol. 7: 862-69, 1995, Li et al., Curr. Biol. 8: 971, 1998, Klug et al., J. Clin. Invest. 98: 216-24, 1996, Lieschke et al., Exp. Hematol. 23: 328-34, 1995, Yamane et al., Blood 90: 3516-23, 1997, and Hirashima et al., Blood 93: 1253-63, 1999.

The particular embryonic stem cell line that is used for genetic modification is not critical; exemplary murine ES cell lines include AB-1 (McMahon and Bradley, Cell 62:1073-85, 1990), E14 (Hooper et al., Nature 326: 292-95, 1987), D3 (Doetschman et al., J. Embryol. Exp. Morph. 87: 27-45, 1985), CCE (Robertson et al, Nature 323: 445-48, 1986), RW4 (Genome Systems, St. Louis, MO), and DBA/1lacJ (Roach et al., Exp. Cell Res. 221: 520-25, 1995). Genetically-modified murine ES cells may be used to generate genetically-modified mice, according to published procedures (Robertson, 1987, *Teratocarcinomas and Embryonic Stem Cells: A Practical Approach,* Ed. E. J. Robertson, Oxford: IRL Press, pp. 71-112, 1987; Zjilstra et al., Nature 342: 435-438, 1989; and Schwartzberg et al., Science 246: 799-803, 1989).

Following confirmation that the ES cells contain the desired functional disruption of the PDE10A gene, these ES cells are then injected into suitable blastocyst hosts for generation of chimeric mice according to methods known in the art (Capecchi, Trends Genet. 5: 70, 1989). The particular mouse blastocysts employed in the present invention are not critical. Examples of such blastocysts include those derived from C57BL6 mice, C57BL6 Albino mice, Swiss outbred mice, CFLP mice, and MFI mice. Alternatively ES cells may be sandwiched between tetraploid embryos in aggregation wells (Nagy et al., Proc. Natl. Acad. Sci. USA90: 8424-8428, 1993).

The blastocysts or embryos containing the genetically-modified ES cells are then implanted in pseudopregnant female mice and allowed to develop *in utero* (Hogan et al., *Manipulating the Mouse Embryo: A Laboratory Manual,* Cold Spring Harbor Laboratory press, Cold Spring Harbor, NY 1988; and *Teratocarcinomas and Embryonic Stem Cells: A Practical Approach,* E.J. Robertson, ed., IRL Press, Washington, D.C., 1987). The offspring born to the foster mothers may be screened to identify those that are chimeric for the PDE10A gene disruption. Generally, such offspring contain some cells that are derived from the genetically-modified donor ES cell as well as other cells derived from the original blastocyst. In such circumstances, offspring may be screened initially for mosaic coat color, where a coat color selection strategy has been employed, to distinguish cells derived from the donor ES cell from the other cells of the blastocyst. Alternatively, DNA from tail tissue of the offspring can be used to identify mice containing the genetically-modified cells.

The mating of chimeric mice that contain the PDE10A gene disruption in germ line cells produces progeny that possess the PDE10A gene disruption in all germ line cells and somatic cells. Mice that are heterozygous for the PDE10A gene disruption can then be crossed to produce homozygotes (see, e.g., U.S. Pat. No. 5,557,032, and U.S. Pat. No. 5,532,158).

An alternative to the above-described ES cell technology for transferring a genetic modification from a cell to a whole animal. is to use nuclear transfer. This method can be employed to make other genetically-modified, non-human mammals besides mice, for example, sheep (McCreath et al., Nature 29: 1066-69, 2000; Campbell et al., Nature 389: 64-66, 1996; and Schnieke et al., Science 278: 2130-33, 1997) and calves (Cibelli et al., Science 280: 1256-58, 1998). Briefly, somatic cells (e.g., fibroblasts) or pluripotent stem cells (e.g., ES-like cells) are selected as nuclear donors and are genetically-modified to contain a functional disruption of the PDE10A gene. When inserting a DNA vector into a somatic cell to mutate the PDE10A gene, it is preferred that a promoterless marker be used in the vector such that vector integration into the PDE10A gene results in expression of the marker under the control of the PDE10A gene promoter (Sedivy and Dutriaux, T.I.G. 15: 88-90, 1999; McCreath et al., Nature 29: 1066-69, 2000). Nuclei from donor cells which have the appropriate PDE10A gene disruption are then transferred to fertilized or parthenogenetic oocytes that are enucleated (Campbell et al., Nature 380: 64, 1996; Wilmut et al., Nature 385: 810, 1997). Embryos are reconstructed, cultured to develop into the morula/blastocyst stage, and transferred into foster mothers for full term *in utero* development.

The present invention also encompasses the progeny of the genetically-modified, non-human mammals and genetically-modified animal cells. While the progeny are heterozygous or homozygous for the genetic modification that disrupts the PDE10A gene, they may not be genetically identical to the parent non-human mammals and animal cells due to mutations or environmental influences, besides that of the original genetic disruption of the PDE10A gene, that may occur in succeeding generations.

The cells from a non-human genetically modified animal can be isolated from tissue or organs using techniques known to those of skill in the art. In one embodiment, the genetically modified cells of the invention are immortalized. In accordance with this embodiment, cells can be immortalized by genetically engineering the telomerase gene, an oncogene, e.g., *mos* or *v-src*, or an apoptosis-inhibiting gene, e.g., *bcl-2,* into the cells. Alternatively, cells can be immortalized by fusion with a hybridization partner utilizing techniques known to one of skill in the art.

### 6. "Humanized" Non-human Mammals and Animal Cells

The genetically-modified non-human mammals and animal cells (non-human) of the invention containing a disrupted endogenous PDE10A gene can be further modified to express the human PDE10A sequence (referred to herein as "humanized"). This includes humanized sequences which contain mutations and/or polymorphisms associated with human disease states. A preferred method for humanizing cells involves replacing the endogenous PDE10A sequence with nucleic acid sequence encoding the human PDE10A sequence (Jakobsson et al., Proc. Natl. Acad. Sci. USA 96: 7220-25, 1999) by homologous recombination. The vectors are similar to those traditionally used as targeting vectors with respect to the 5' and 3' homology arms and positive/negative selection schemes. However, the vectors also include sequence that, after recombination, either substitutes the human PDE10A coding sequence for the endogenous sequence, or effects base pair changes, exon substitutions, or codon substitutions that modify the endogenous sequence to encode the human PDE10. Once homologous recombinants have been identified, it is possible to excise any selection-based sequences (e.g., neo) by using Cre or Flp-mediated site directed recombination (Dymecki, Proc. Natl. Acad. Sci. 93: 6191-96, 1996).

When substituting the human PDE10A sequence for the endogenous sequence, it is preferred that these changes are introduced directly downstream of the endogenous translation start site. This positioning preserves the endogenous temporal and spatial expression patterns of the PDE10A gene. The human sequence can be the full length human cDNA sequence with a polyA tail attached at the 3' end for proper processing or the whole genomic sequence (Shiao et al., Transgenic Res. 8: 295-302, 1999). Further guidance regarding these methods of genetically modifying cells and non-human mammals to replace expression of an endogenous gene with its human counterpart is found, for example, in Sullivan et al., J. Biol. Chem. 272: 17972-80, 1997, Reaume et al., J. Biol. Chem. 271: 23380-88, 1996, and Scott et al., U.S. Pat. No. 5,777,194).

Another method for creating such "humanized" organisms is a two step process involving the disruption of the endogenous gene followed by the introduction of a transgene encoding the human sequence by pronuclear microinjection into the knock-out embryos.

### 7. Uses for the Genetically-Modified Non-human Mammals and Animal Cells

PDE10A function and therapeutic relevance can be elucidated by investigating the phenotype of the non-human mammals and animals cells of the invention that are homozygous (-/-) and heterozygous (+/-) for the disruption of the PDE10A gene. For example, the genetically-modified PDE10A -/- non-human mammals and animal cells can be used to determine whether the PDE10A plays a role in causing or preventing symptoms or phenotypes to develop in certain models of disease, e.g., neuropsychiatric disorder and neurodegeneration models. If a symptom or phenotype is different in a PDE10A -/- non-human mammal or animal cell as compared to a wild type (PDE10A +/+) or PDE10A +/- non-human mammal or animal cell, then the PDE10A polypeptide plays a role in regulating functions associated with the symptom or phenotype. Examples of animal models that can be used to assess PDE10A function by comparing PDE10A -/- mice to wild type mice include models of locomotor activity, psychostimulant response, learning/memory, behavioral modification (e.g., fear response), and neurodegeneration.

In addition, under circumstances in which an agent has been identified as a PDE10A agonist or antagonist (e.g., the agent significantly modifies one or more of the PDE10A polypeptide activities when the agent is administered to a PDE10A +/+ or PDE10A +/- non-human mammal or animal cell), the genetically-modified PDE10A -/- non-human mammals and animal cells of the invention are useful to characterize any other effects caused by the agent besides those known to result from the (ant)agonism of PDE10A (i.e., the non-human mammals and animal cells can be used as negative controls). For example, if the administration of the agent causes an effect in a PDE10A +/+ non-human mammal or animal cell that is not known to be associated with PDE10A polypeptide activity, then one can determine whether the agent exerts this effect solely or primarily through modulation of PDE10A by administering the agent to a corresponding PDE10A -/- non-human mammal or animal cell. If this effect is absent, or is significantly reduced, in the PDE10A -/- non-human mammal or animal cell, then the effect is mediated, at least in part, by PDE10A. However, if the PDE10A -/- non-human mammal or animal cell exhibits the effect to a degree comparable to the PDE10A +/+ or PDE10A +/- non-human mammal or animal cell, then the effect is mediated by a pathway that does not involve PDE10A signaling.

Furthermore, if an agent is suspected of possibly exerting an effect via a PDE10A pathway, then the PDE10A -/- non-human mammals and animal cells are useful to test this hypothesis. If the agent is indeed acting through PDE10A, then the PDE10A -/- non-human mammals and animal cells, upon administration of the agent, should not demonstrate the same effect observed in the PDE10A +/+ non-human mammals or animal cells.

The PDE10A -/- non-human mammals of the invention are also useful to cross with other genetically modified non-human mammals or other models of disease, e.g., mice expressing proteins which cause them to exhibit a phenotype similar to Huntington's Disease in humans.

The genetically modified non-human mammals and animal cells of the invention can also be used to identify genes whose expression is upregulated in PDE10A +/- or PDE10A -/- non-human mammals or animal cells relative to their respective wild-type control. Techniques known to those of skill in the art can be used to identify such genes based upon the present description. For example, DNA assays can be used to identify genes whose expression is upregulated in PDE10A +/- or PDE10A -/- mice to compensate for a deficiency in PDE10A expression. DNA arrays are known to those of skill in the art (see, e.g., Aigner et al., Arthritis and Rheumatism 44: 2777-89, 2001; U.S. Patent No. 5,965,352; Schena et al., Science 270: 467-470, 1995; DeRisi et al., Nature Genetics 14: 457-460, 1996; Shalon et al., Genome Res. 6: 639-645, 1996; and Schena et al., Proc. Natl. Acad. Sci. (USA) 93: 10539-11286, 1995).

The examples below are provided to illustrate the subject invention and are not included for the purpose of limiting the invention.

### Examples

### A. Targeting Vector Construction

A 2.2 kb genomic fragment was used to hybridize a DBA/1lacJ genomic lambda phage library (Stratagene, La Jolla CA). This genomic fragment contained base pairs 1898-2146 of the murine PDE10A cDNA sequence (Genbank AF110507) and an intron of approximately 2.0 kb. Three overlapping PDE10A genomic clones were isolated and subcloned into the Not I site of pBluescript SK+ (Stratagene, La Jolla, CA). These clones were restriction mapped and determined to contain 22 kb of the PDE10A genomic locus including 5 exons (base pairs 1484-2303 of the cDNA).

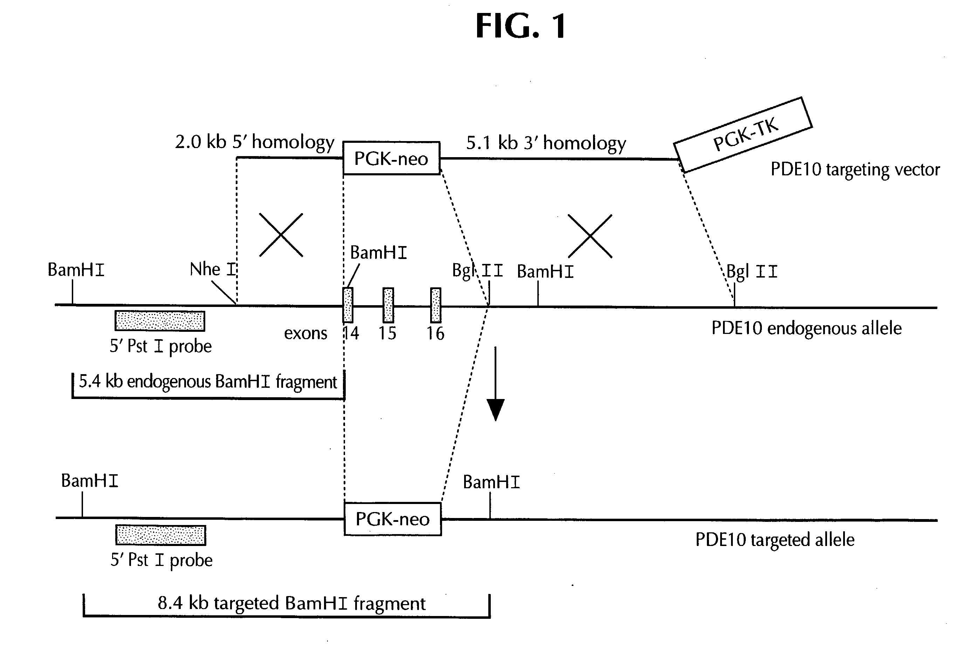

A 2.0 kb Nhe I / BamHI fragment was isolated and cloned intothe Xba I / BamHI sites of pBluescript SK+. The BamHI restriction site was destroyed in the resultant clone by doing a Klenow (Roche Diagnostics, Indianapolis IN) fill-in reaction. The 2.0 kb fragme was re-isolated from the pBluescript SK+ vector with a Not I / Xho I digest and cloned into the Not I / Xho I sites of the pJNS2-Frt targeting vector backbone (Dombrowicz et al., Cel 75: 969-76, 1993) to serve as the 5' homology arm. The 3' homology arm was isolated a a 5.1 kb Bgl II fragment. This 5.1 kb fragment was cloned into the BamHI cloning site of the pJNS2-Frt targeting vector (which already contained the 5' homology arm) to produce the complete targeting vector. The targeting vector was designed to replace 3.9 kb of the PDE10A genomic locus with the PGK-neomycin cassette (Fig. 1); the deleted 3.9 kb genomic fragment contains 3 exons encoding base pairs 1499- 1861 of the cDNA sequence.

### B. ES Cell Screening

The PDE10A targeting vector was linearized with Notl and electroporated into DBA/1LacJ ES cells (Roach et al., Exp. Cell. Res. 221: 520-25, 1995). Pluripotent ES cells were maintained in culture on a mitomycin C treated primary embryonic fibroblast (PEF) feeder layer in stem cell medium (SCML) which consisted of knockout DMEM (Invitrogen Life Technologies, Inc., (ILTI) Gaithersburg, MD, #10829-018) supplemented with 15% ES cell qualified fetal calf serum (ILTI, #10439-024), 0.1 mM 2-mercaptoethanol (Sigma, St. Louis, MO, #M-7522), 0.2 mM L-glutamine (ILTI, #25030-081), 0.1 mM MEM non-essential amino acids (ILTI, #11140-050), 1000 units/ml recombinant murine leukemia inhibitory factor (Chemicon International Inc., Temecula, CA, Catalog No. ESG-1107), and penicillin/streptomycin (ILTI, #15140-122).

Electroporation of 1X10⁷ cells in SCML and 25 µg linearized targeting vector was carried out using a BTX Electro Cell Manipulator 600 (BTX, Inc., San Diego, CA) at a voltage of 240 V, a capacitance of 50 µF, and a resistance of 360 Ohms. Positive/negative selection began 24 hours after electroporation in SCML which contained 200 µg/ml G418 (ILTI, #11811-031) and 2 µM gancyclovir (Syntex Laboratories, Palo Alto, CA), as previously described (Mansour et al, Nature 236: 348-52, 1988).

Resistant colonies were picked with a micropipette following 8-12 days of selection. Expansion and screening of resistant ES cell colonies was performed as described in Mohn and Koller (Mohn, *DNA Cloning* 4 (ed. Hames), 143-184, Oxford University Press, New York, 1995).

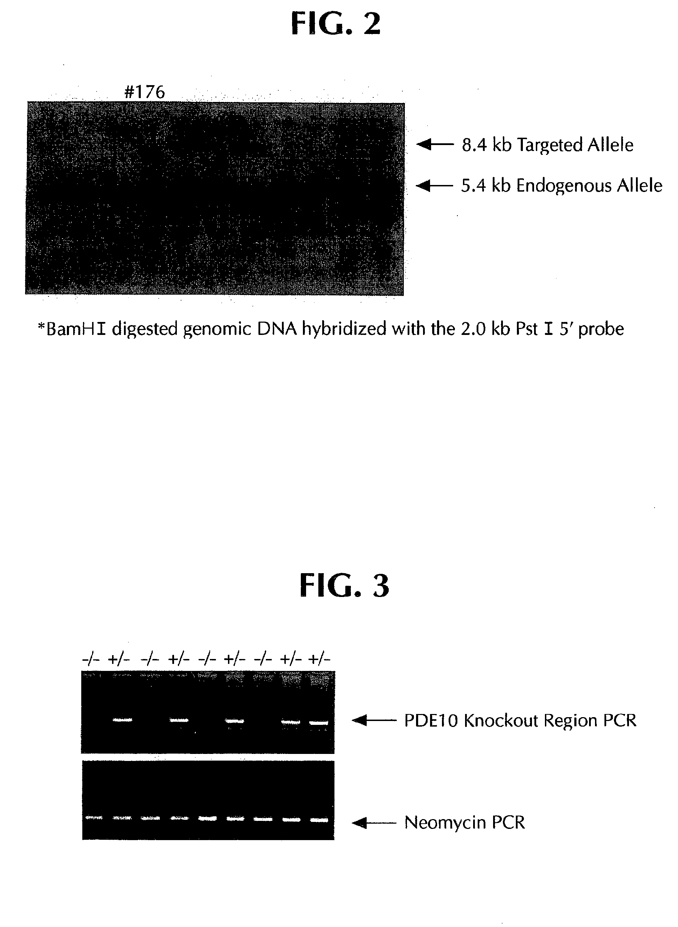

DNA was isolated from 130 ES cell clones which survived G418 and gancyclovir selection. The DNA was digested with BamHI and electrophoresed on 0.7% agarose gels (BioWhittaker Molecular Applications, Rockland ME) and transferred to Hybond N+ nylon membrane (Amersham Pharmacia Biotech, Buckinghamshire England) for Southern analysis. A 2.2 kb Pst I genomic fragment, upstream of the 5' homology arm was used as a probe to identify homologously recombined ES cells. This 2.0 kb probe recognizes a 5.4 kb endogenous allele and a 8.4 kb targeted allele due to the loss of an endogenous BamHI site in the targeted allele. A single targeted clone (clone #176) was identified using the 5' Pst I probe (Fig. 2). Targeting on the 3' side for this clone was confirmed using a 1.2 kb Nhe I / EcoRV fragment as a probe. This fragment is downstream of the 3' homology arm and recognizes a 8.0 kb endogenous EcoRV / Nhe I fragment and a 5.7 kb fragment for a targeted allele due to a newly introduced EcoRV site within the neomycin cassette in the targeted allele.

### C. Knockout Mouse Production

ES cells from clone #176 were microinjected into blastocyst stage embryos isolated from C57BL/6J females (The Jackson Laboratory, Bar Harbor ME). Male chimeras were identified and back-crossed to DBA/1lacJ females (The Jackson Laboratory) to derive germline PDE10A heterozygous (+/-) offspring.

Heterozygous animals were genotyped by PCR for the presence of the neomycin cassette. The primer set consisted of Neo-833F (5' gcaggatctcctgtcatctcacc 3') (SEQ ID NO: 1) and Neo-1023R (5' gatgctcttcgtccagatcatcc 3') (SEQ ID NO: 2). This oligo set amplified a 190 bp fragment from a targeted PDE10A allele.

Heterozygous males and females were mated to generate homozygous (-/-) PDE10 knockouts. These offspring were genotyped using a 2 primer set approach, one specific for the neomycin cassette (Neo-833F and Neo-1023R) and the second set specific for the PDE10 knockout region (Fig. 3). The PDE10A specific primer set amplified a 613 bp fragment contained within the 3.9 kb knockout region and consisted of PDE10KO-649F (5' ggctgctgcaacatgagtgtcc 3') (SEQ ID NO: 3) and PDE10KO-1262R (5' actgaggtgtttactgtctgttcc 3') (SEQ ID NO: 4). Normal Mendelian ratios were observed in offspring from these matings. For example, from 32 litters, 50 -/-, 92 +/-, and 42 +/+ offspring were obtained.

### D. Characterization of PDE10A RNA in PDE10 KO mice.

The cDNA isolated from -/- mouse brain contained a deletion of 378 nucleotides as compared to wildtype DNA, based on genomic organization of the PDE10A gene (Fujishige et al (2000) Eur. J. Biochem. 267: 5943-5951), was generated by a splicing event that joined the 3' end of exon 15 to the 5' end of exon 19. This deleted cDNA coded for an in-frame PDE10A fragment with a predicted molecular weight of approximately 75 kDa that did not include 126 amino acids at the end of the N-terminal region of the catalytic domain in comparison to the wild type protein.

Total RNA was isolated from wild type and KO mouse striatum using RNAeasy (Qiagen) and first strand cDNA was synthesized using first Strand cDNA Synthesis Kit (Invitrogen) and oligo dT primer. PCR was carried out using Robocycler (Stratagene) with primers designed to the catalytic region of PDE10 downstream of the deletion/insertion generated in the KO (5' primer: ggaccacaggggcttcagtaacag; (SEQ ID NO: 5) 3' primer: tgcccagcttcttcatctcatcac (SEQ ID NO: 6), Taq Polymerase (Roche) and the following cycling conditions: 94°C for 3 min, then 35 cycles of 30 sec at 94°C, 30 sec at 55°C, and 3 min 30 sec at 68°C, followed by a final extension period of 5 min at 68°C. Unexpectedly, the results showed a 428 bp product in RNA isolated from both PDE10A +/+ and -/- mouse brain, suggesting that RNA containing catalytic domain sequences was present in both PDE10A +/+ and -/- mouse brain. To characterize the RNA species in the -/- animal, we isolated cDNA from brain of PDE10A -/- mice using first strand cDNA prepared as above and PDE10A PCR primers that contained a 5' BAMHI site (5' primer: tggatcctataaatatggaaaattatatggtttgacggatgaaaagg) (SEQ ID NO: 7) and a 3' Sall site (3' primer: attatgtcgacgtcttcaaccttcacgttcag) (SEQ ID NO: 8). PCR was performed on a Robocycler using Pfu Polymerase (Stratagene) and the following sycling conditions: initial 94°C for 2 min, then 4 cycles of 1 min at 94°C, 1 min at 48°C, and 4 min at 72°C, followed by 30 cycles of 1 min at 94°C, 1 min at 55°C, and 4 min at 72°C, followed by a final extension at 72°C for 10 min. The approximately 1980 bp product was subcloned into pCR Bluntll TOPO (Invitrogen) and sequenced.

### E. Loss of PDE10A Activity in PDE10 KO Mice.

Western Analysis. The genomic deletion/insertion generated in the KO mouse results in a loss of expression of full length PDE10A protein and of PDE10A enzymatic activity in PDE10A +/- and -/- mice, as illustrated in the example below. PDE10A protein and activity were analyzed in extracts of the striatum of the genetically modified mice. This brain region was chosen since it is the area of highest PDE10A expression in mammals. In some cases, cortical extracts were also analyzed for comparison, since cortex has comparatively very much less PDE10A expression.

PDE10A +/+, +/-, or -/- mice were euthanized, brains were rapidly removed and striatum and cortex obtained by microdissection. Tissue was homogenized in a glass-teflon dounce homogenizer in buffer contained 50mM Tris-HCI, pH 7.5, 250mM NaCI, 5mM EDTA, pH 8.0, 0.1% nonidet P-40 and protease inhibitors. The lysates were centrifuged for 30 min. at 4°C. The pellets were discarded and the supernatant lysates were stored at -80°C. Protein concentrations were determined by Pierce BCA method.

PDE10A protein expression was analyzed by Western blotting with a monoclonal antibody (24F3.F11) to the N-terminal region of PDE10A (Menniti, F.S., Strick, C.A., Seeger. T.F., and Ryan, A.M. William Harvey Research Conference 'Phosphodiesterase in Health and Disease', Porto, Portugal, Dec 5-7, 2001.) Equal amounts of protein from PDE10A +/+, +/-, or -/- mice striatal lysates were resolved on 4-12% NuPAGE Bis-Tris gel (Novagen) in MOPS running buffer and transferred to nitrocellulose. The blots were incubated with anti-PDE10A monoclonal antibody at 1:1000-1:2000 dilution, followed by incubation with horse radish-peroxidase conjugated sheep anti-mouse secondary antibody (Amersham). The blots were developed using the ECL Detection System (Amersham).

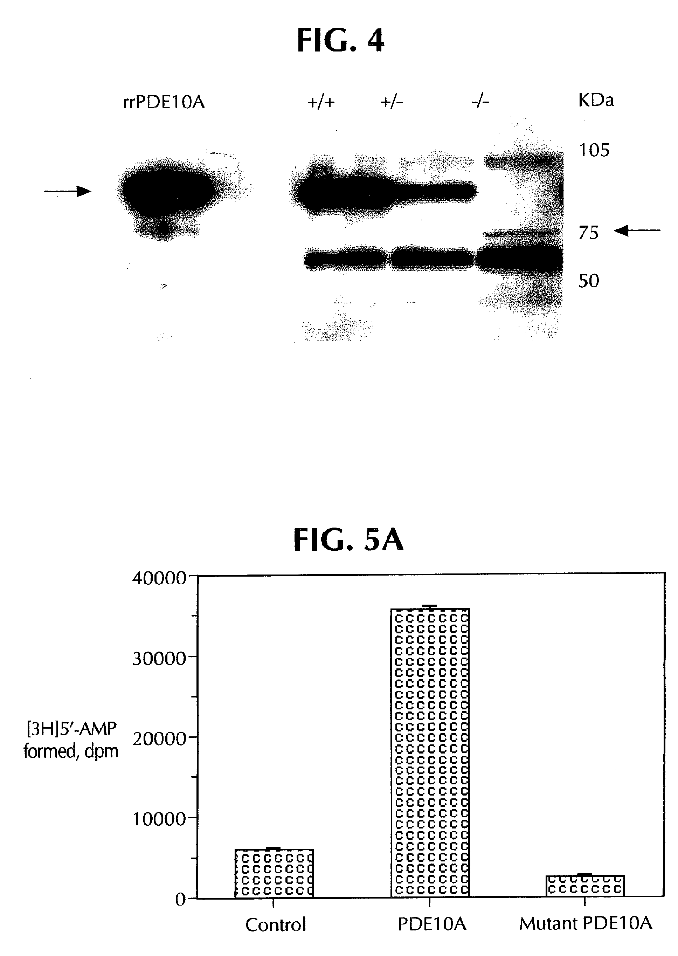

As indicated in Figure 4, a band of immunoreactivity is observed at molecular weight range of approximately 89 kDa for purified recombinant PDE10A (lane 1 left arrow). In striatal extract from PDE10A +/+ mice (lane 2), a band in this molecular weight range is also observed indicating the presence of PDE10A protein. However, this band is significantly less intense in an equal amount of striatal extract from PDE10A +/- mice (lane 3) and no band is observed in extract from PDE10A -/- animals (lane 4). Interestingly, the antibody recognizes a new band of the approximate molecular weight predicted for the potential protein coded by the deleted PDE10A RNA in striatal extract from PDE10A -/- mice (right arrow). Recombinant Expression and Activity of PDE10A from -/- Mice.

To ensure that the mutant PDE10A mRNA present in the -/- mice did not produce an active protein, the cDNA isolated from -/- mice was subcloned as a BamHI/Sall fragment into pFastBacl (Invitrogen) and used to generate virus for expression in insect cells following the manufacturer's protocol (Bac-to-Bac, Invitrogen). Sf9 cells were infected with virus encoding full length PDE10A cDNA or the deletion mutant cDNA and then cell extracts were assayed for phosphodiesterase activity. Transfected SF9 insect cells were seeded in suspension culture at 0.5 X 10⁶/ml in SF900II-SFM (Invitrogen) and grown overnight at 120 rpm, 27°C. Cells were infected at an MOI of approximately 1 and harvested after 72 hrs. Cells were homogenized in 50 mM TRIS buffer (pH 7.4) containing protease inhibitors by sonication. The homogenate was centrifuged at 14,000 x g for 20 min and the resultant supernatant used for enzyme assay.

PDE activity in cell extracts was measured using a Scintillation Proximity Assay (SPA)-based method as previously described (Fawcett et al., 2000). Different amounts of cell extract were added to assay buffer [20 mM Tris-HCI pH 7.4, 5 mM MgCl2, 1 mg/ml bovine serum albumin] containing 20 uM [3H]cAMP in individual wells of a 96-well microtiter plate (100 ul, final volume) and incubated for 20-30 min at room temperature or 30°C. Reactions were terminated with 50 ul yttrium silicate SPA beads (Amersham). Plates were sealed and shaken for 20 min, after which the beads were allowed to settle fo 30 min in the dark and then counted on a TopCount plate reader (Packard, Meriden, CT) Increases in enzyme activity is indicated by an increase in SPA detected [3H]5'-AMP, which is the product of phosphodiesterase catatlyzed breakdown of [3H]cAMP, at different dilution of cell extracts.'

Homogenates of untransfected Sf9 cells have a low level of endogenous PDE activity (Fig. 1A). PDE activity is increased more than 50-fold in homogenates of Sf9 cells transfected with full length PDE10A cDNA. However, in homogenates of Sf9 cells transfected with PDE10A in which bases 1499-1861 have been deleted, the PDE activity was indistinguishable from that in untransfected cells. The disruption generates a catalytically-inactive endogenous PDE10A protein. Thus, the PDE10A mRNA present in PDE10A -/- mice is not capable of generating a catalytically-active protein.

PDE10A Activity in Immunoprecipitated Proteins from PDE10A +/+ and -/-Mice.

The apparent lack of PDE10A expression in PDE10A -/- mice was confirmed by isolating PDE10A protein from striatal extracts by immunoprecipitation and assaying for enzymatic activity. For immunoprecipitation, a slurry of washed protein A/G beads was incubated with antibody 24F3.F11 for 2 h at 22°C. The bead/antibody complex was washed and then incubated with protein extract from PDE10A +/+, +/-, or -/- mice (1:10 ratio of beads to protein). This mixture was incubated for a further 2h at 22°C while being constantly stirred by rotation. The protein A/G beads were pelleted by centrifugation and washed with PBS. This immunopercipitate complex was then assayed for phosphodiesterase activity in the SPA assay described above.

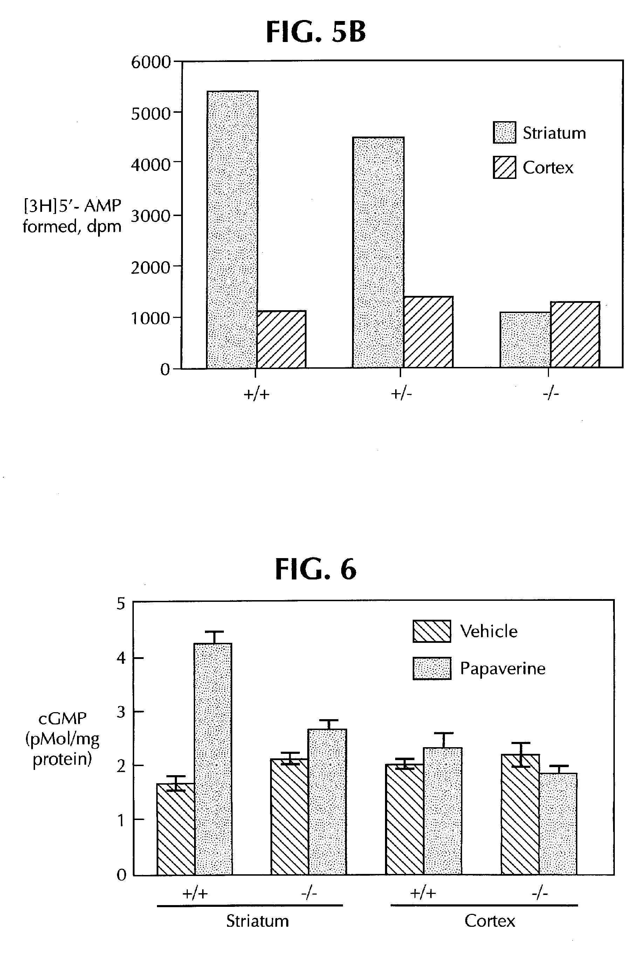

As indicated in Figure 5, significant phosphodiesterase activity was observed in immunoprecipitates from striatal extract from PDE10A +/+ mice. However, the amount of activity recovered from extracts of PDE10A +/- mice was reduced relative to that in extracts from wild type mice and an even greater reduction was observed relative to that shown in extracts from PDE10A -/- mice. In all three groups of animals, phosphodiesterase activity was also observed in the immunoprecipitates from cortical tissues; however, the level of this activity did not change across the different groups and is presumed to be phosphodiesterase nonspecifically extracted in the immunoprecipitation reaction. It is noteworthy that the level of phosphodiesterase activity is similar in striatal and cortical extracts from the PDE10A -/- mice. This suggests that the activity observed in the striatal extracts from the PDE10A -/- activity is not likely due to residual PDE10A activity but to other nonspecifically extracted phsophodiesterase activity. These data, coupled with the data from Western blot analyses and recombinant expression of the deletion PDE10A protein described above, indicate that the PDE10 -/- mice have a very substantial reduction or absence of expression of PDE10A protein and enzymatic activity.

### F. Loss of PDE10A Inhibitor Activities in PDE10A -/- Mice.

PDE10A knockout mice are useful for determining if an agent is exerting a biological effect via interaction with PDE10A. If the agent is indeed acting through PDE10A, then the PDE10A -/- mice, upon administration of the agent, should not demonstrate the same effect observed in the PDE10A +/+ animals. This is illustrated in the following example of the effect of the PDE10A inhibitor Papaverine (#76223 Sigma-Aldrich, St. Louis, MO) on changes in striatal cGMP level. PDE10A +/+ and -/- mice were administered intraperitoneally 32 mg/kg Papaverine or vehicle. Fifteen minutes afterwards, mice were euthanized by focused-beam microwave irradiation of the head (2 second exposure in a Litton Systems 70/50 microwave, model BN-K2, General Medical Engineering Corp., Peabody, MA, USA) to prevent enzymatic degradation of cyclic nucleotides. Striatal and cortical regions were isolated and stored at -80°C until analysis. Samples were homogenized in 0.5 N HCI and then centrifugation at 20,000 x g for 30 minutes. The cGMP concentrations in the supematants were determined using enzyme immunoassay kits (Cayman Chemical, Ann Arbor, Ml, USA) according to the manufacturer's instructions.

Papaverine, relative to vehicle, caused a more than two fold elevation in the cGMP level from striatal extracts from PDE10A +/+ mice (Figure 6). There was no effect of Papaverine relative to vehicle in cGMP level in cortical extracts from these animals. This differential effect of Papaverine in striatum vs. cortex reflects the high level of expression of PDE10A in striatum relative to cortex. In contrast to the PDE10A +/+ animals, Papaverine caused only a slight increase in striatal cGMP level in tissues from the PDE10A -/- mice. This observation is predicted from the significant reduction in PDE10A protein and enzymatic activity detected in the PDE10A -/- animals described above.

A further example of the utility of the PDE10A knockout mice for determining if an agent is exerting a biological effect via interaction with PDE10A is illustrated in an assay of the effect of the PDE10A inhibitor Papaverine on conditioned avoidance responding. In the conditioned avoidance paradigm, animals are trained to move from a dark chamber in a shuttle box to a lighted chamber upon presentation of a conditioned stimulus in order to avoid a foot shock. Agents shown to have antipsychotic activity in man have been found to inhibit this conditioned avoidance response and the ability of novel compounds to inhibit this response is thought to be predictive of antipsychotic efficacy in man (Wadenberg, M.-L.G. and Hicks, P.B., *Neurosci. Biobehav. Rev.* 23:851-862, 1999).

The conditioned avoidance shuttle chambers consist of individual Plexiglas behavior chambers (Coulbourn Instruments) each divided by a guillotine door into two sides, enclosed in sound attenuating cabinets. The Plexiglas chambers are fitted with metal grid floors, which are equipped with scrambled/constant current shockers. Mice are trained to avoid the onset of footshock (0.6 miliampere) by moving to the opposite side of the chamber upon activation of house lights, que lights, and the opening of the guillotine door, all of which occur 5 seconds prior to the foot shock. A computer program is used to record the number of successful avoidance responses (animals cross before onset of shock), as well as the number of escapes (where animals receive the foot shock and then cross), escape failures (where animals receive the foot shock yet still fail to cross), and the latencies to avoid (max 5 sec.) or escape (max.10 sec.). Thirty trials are completed per daily session, and Inter-trial intervals are 15 seconds with the guillotine door closed. Drug treatment sessions begin when mice have reached criteria of 80% avoidances for a session. Vehicle treatment is performed one day every week and statistical analysis is done comparing each drug treatment on separate days vs. the vehicle treatment that week. Testing is performed during the lights on period of the light/dark cycle, typically between 8am and 11am. The data is analyzed using a paired t-test with each animal as it's own control.

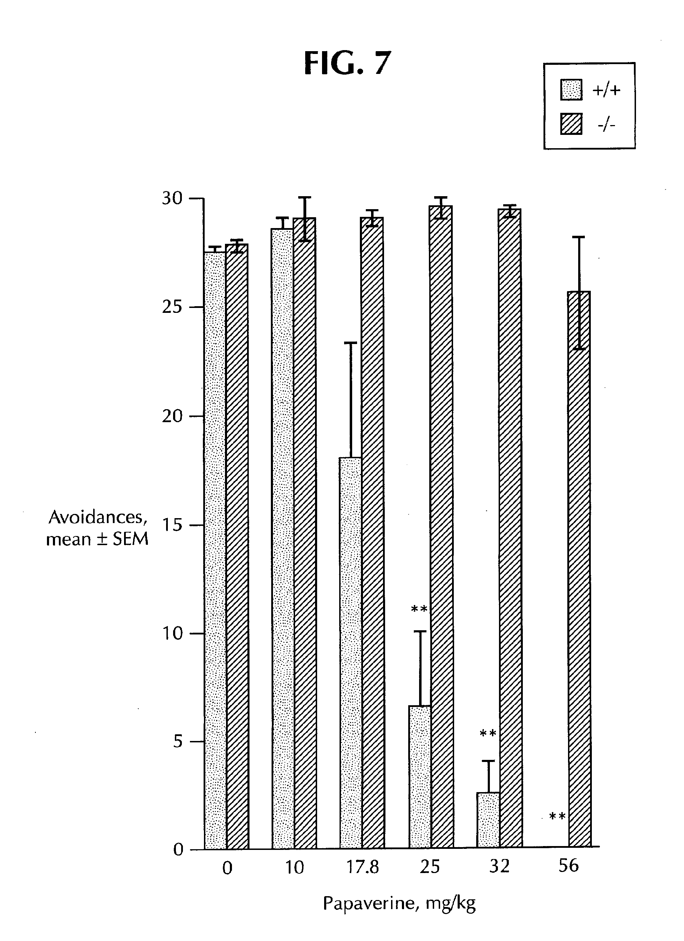

The PDE10A +/+ and -/- mice both learned the conditioned avoidance response to criteria in a similar number of learning trials. The known antipsychotic agent Haloperidol (#H1512, Sigma-Aldrich, St. Louis, MO) inhibited conditioned avoidance responding in both groups of mice with similar potency (ED50 of 0.21 mg/kg in PDE10A +/+, and ED50 of 0.29 mg/kg in PDE10A -/- mice; Wilcoxan Signed Rank Test, p > 0.05). In the PDE10A +/+ mice, Papaverine also inhibited conditioned avoidance responding with an ED50 of 20.7 mg/kg (Figure 7). At the highest dose tested (56 mg/kg) there was no effect of Papaverine on escape failures, indicating that the effect of this compound on conditioned avoidance responding is not due to the induction of ataxia. In contrast, Papaverine at doses up to 56 mg/kg failed to inhibit conditioned avoidance responding in the PDE10A -/mice. This example indicates that the effect of Papaverine on conditioned avoidance responding is mediated through an interaction with PDE10A.

## Claims

1. A genetically-modified, non-human mammal, wherein the modification results in a disrupted phosphodiesterase 10A (PDE10A) gene.

2. The mammal of claim 1, wherein said mammal is a rodent and preferably a mouse.

3. The non-human mammal of claim 1, wherein said disruption generates a catalytically-inactive endogenous PDE10A protein.

4. A genetically-modified animal cell, wherein the modification comprises a disrupted PDE10A gene.

5. The animal cell of claim 4, wherein said cell is an embryonic stem (ES) cell or an ES-like cell.

6. The animal cell of claim 4, wherein said cell is isolated from a genetically-modified, non-human mammal containing a modification that results in a disrupted PDE10A gene.

7. The animal cell of claim 6, wherein said cell is neuronal.

8. The animal cell of claim 4, wherein said cell is murine or human.

9. A method of identifying a gene that demonstrates modified expression as a result of reduced PDE10A activity in an animal cell, said method comprising assessing the expression profile of an animal cell containing a genetic modification that results in reduced PDE10A polypeptide levels in said cell, and comparing said profile to that from a wild type cell.

10. A screening assay for evaluating whether a compound is effective as a PDE10A agonist or antagonist which comprises:
(a) administering the compound to the genetically-modified nonhuman mammal of claim 1 wherein the mammal has decreased PDE10A activity, and
(b) comparing the effect of said compound on the mammal in step (a) with a genetically-modified mammal having decreased PDE10A activity in the absence of the compound or with a mammal having PDE10A activity so as to determine whether the compound is effective as an agonist or antagonist of PDE10A activity.

11. A screening method as recited in claim 18 wherein said assays are utilized to assess PDE10A functions in one or more of locomotor response activity, psychostimulant response, learning/memory, behavior modification or neurodegeneration.


SEQUENCE LISTING

<110>  Pfizer Products Inc.

<120>  DISRUPTION OF THE PHOSPHODIESTERASE 10 GENE

<130>  PC23257A

<150>  60/343,479
<151>  2002-12-21

<160>  8

<170>  PatentIn version 3.1

<210>  1
<211>  23
<212>  DNA
<213>  mus musculus

<400>  1
gcaggatctc ctgtcatctc acc                                              23

<210>  2
<211>  23
<212>  DNA
<213>  mus musculus

<400>  2
gatgctcttc gtccagatca tcc                                              23

<210>  3
<211>  22
<212>  DNA
<213>  mus musculus

<400>  3
ggctgctgca acatgagtgt cc                                               22

<210>  4
<211>  24
<212>  DNA
<213>  mus musculus

<400>  4
actgaggtgt ttactgtctg ttcc                                             24

<210>  5
<211>  24
<212>  DNA
<213>  mus musculus

<400>  5
ggaccacagg ggcttcagta acag                                             24

<210> 6
<211> 24
<212> DNA
<213> mus musculus

<400> 6
tgcccagctt cttcatctca tcac 24

<210> 7
<211> 47
<212> DNA
<213> mus musculus

<400> 7
tggatcctat aaatatggaa aattatatgg tttgacggat gaaaagg 47

<210> 8
<211> 32
<212> DNA
<213> mus musculus

<400> 8
attatgtcga cgtcttcaac cttcacgttc ag 32